(19) **Europäisches Patentamt**

**European Patent Office**

**Office européen des brevets**

(11) Veröffentlichungsnummer: **0 235 673**
**A1**

# (12) EUROPÄISCHE PATENTANMELDUNG

(21) Anmeldenummer: **87102269.5**

(22) Anmeldetag: **18.02.87**

(51) Int. Cl.⁴: **A61B 10/00**

(30) Priorität: **03.03.86 CH 857/86**

(43) Veröffentlichungstag der Anmeldung:
**09.09.87 Patentblatt 87/37**

(84) Benannte Vertragsstaaten:
**AT BE DE ES FR GB IT LU NL SE**

(71) Anmelder: **Graf, Ernst**
**Aachweg 3a**
**CH-9323 Steinach(CH)**

(72) Erfinder: **Graf, Ernst**
**Aachweg 3a**
**CH-9323 Steinach(CH)**

(74) Vertreter: **Römpler, Aldo et al**
**Patentanwälte Georg Römpler und Aldo**
**Römpler Schützengasse 34 Postfach 148**
**CH-9410 Heiden(CH)**

(54) **Spatel für die zytologische Abstrichnahme.**

(57) Der bleistiftförmige Handgriffteil (1) hat einen kreisrunden Querschnitt und eine rauhe Oberfläche. Der Abstrichteil weist die Form von zwei voneinander gespreizten Fingern (2, 3) auf, von denen der eine Finger (2) länger und schmäler ist als der andere Finger (3). Der Abstrichteil mitsamt den Fingern (2, 3) ist flach ausgebildet. Eine der beiden Flächen des Abstrichteils weist eine Körnung (4) auf; diese ist kantenlos, halbkugelförmig bis halboval ausgebildet und hat eine gleichmässige Höhe von annähernd 1/10 mm. Die sich gegenüber liegenden Kanten der Finger (2, 3) und der Übergang von Finger zu Finger sind abgerundet und mit einer Körnung versehen. Die übrigen Kanten sind zwar abgerundet, besitzen aber keine Körnung. Die ungekörnte Fläche des Abstrichteils ist mit einer Stabilisierungsrippe (5) versehen. Der längere Finger (2) weist eine gegen den anderen Finger gerichtete polsterartige Abrundung (6) auf. Die Zell-Zahl im Abstrich ist um ein Vielfaches höher als bei bekannten Spateln.

FIG. 1

EP 0 235 673 A1

## "Spatel für die zyytologische Abstrichnahme"

Spatel für die zytologische Abstrichnahme sind in Form von Holzspateln und Entnahmetupfern, insbesondere bei der gynäkologischen Zytodiagnostik bekannt.

Die frühe Erkennung eines Karzinoms wird in hohem Masse von der Qualität der Zellgewinnungs-und Zellübertragungsmethode beeinflusst. In umfangreichen Untersuchungen über die Effizienz herkömmlicher Zellabstrichverfahren wurde nachgewiesen, dass bei der Anwendung der Wattetupfertechnik im Verlauf der nachfolgenden zytologischen Untersuchung nur 20% der Zellveränderungen entdeckt werden können. Von der ohnehin geringen Zellmenge, die mit dieser Technik gewonnen werden kann bleibt ein hoher Anteil mit den Tupfer-Fasern verbunden, so dass nur ein Fünftel des wenigen abgestrichenen Zellmaterials auf den Objektträger übertragen wird. Das kann zu einer Erhöhung falsch beurteilter Präparate führen.

Die Erfindung bezweckt ein Spatel zu schaffen, der die Nachteile bekannter Spatel und Wattetupfer nicht aufweist.

Der erfindungsgemässe Spatel entspricht den kennzeichnenden Merkmalen des Patentanspruchs 1.

Nachfolgend wird anhand der Zeichnungsfiguren ein Ausführungsbeispiel des erfindungsgemässen Spatels beschrieben, und zwar eines Spatels für die zervixcitologische Abstrichnahme und Lokalisation von intrazervikalen Karzinomen.

Fig. 1 zeigt einen Spatel in Vorderansicht und in Rückansicht,

Fig. 2 zeigt den Abstrichteil des Spatels während der Abstrichnahme, und

Fig. 3 zeigt die Körnung auf dem Spatel in fünfzigfacher Vergrösserung.

Der bleistiftförmige Handgriffteil 1 des Spatels hat einen kreisrunden Querschnitt und eine rauhe Oberfläche, damit sich der Spatel während der Abstrichnahme leicht mittels zwei Fingern um seine Achse drehen lässt.

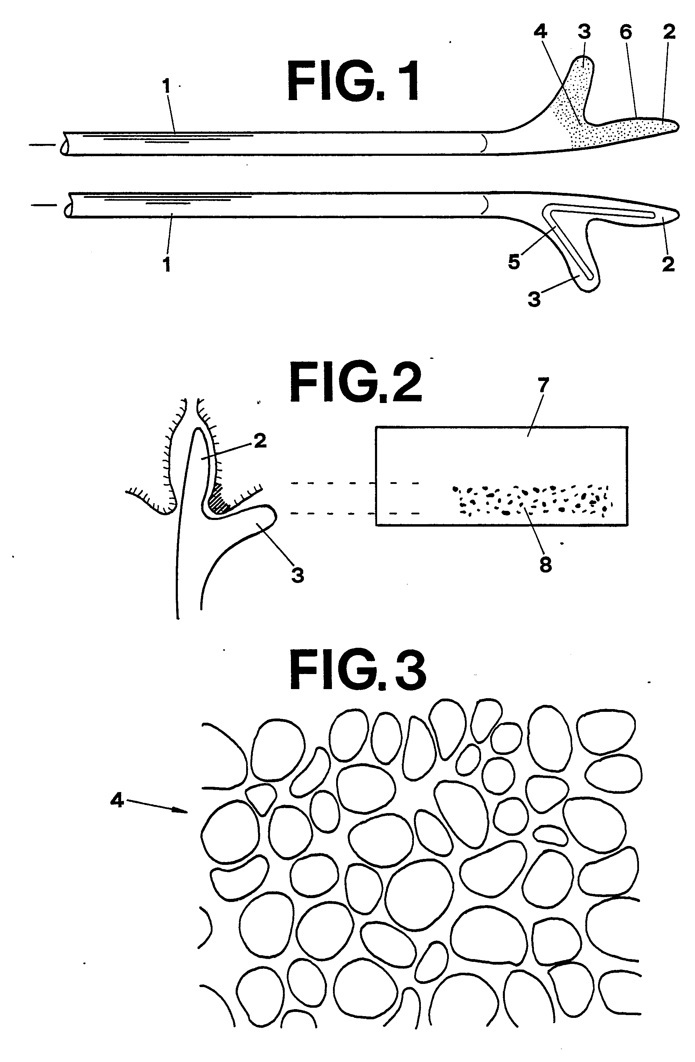

Der Abstrichteil weist die Form von zwei Fingern 2 und 3 auf, die wie Zeigefinger und Daumen im Winkel voneinander gespreizt sind. Der annähernd in Spatel-Längsrichtung zeigende Finger 2 ist länger und schmäler als der andere Finger 3. Der längere Finger 2 weicht in einem Winkel von 7° von der Spatel-Längsrichtung ab. Der Abstrichteil mitsamt den Fingern 2 und 3 ist flach ausgebildet. Bei nach links gerichtetem kleinen Finger 3 weist die oberseitige Fläche des Abstrichteils mit den Fingern 2 und 3 eine Körnung 4 auf, die untenseitige Fläche jedoch nicht. Diese Körnung 4 ist in Fig. 3 in fünfzigfacher Vergrösserung dargestellt; sie ist kantenlos, halbkugelförmig bis halboval ausgebildet, hat eine gleichmässige Höhe von annähernd 1/10 mm und ist unregelmässig angeordnet. Die jeweils gegen den anderen Finger gerichteten Kanten der Finger 2 und 3, sowie auch die Kanten des Übergangs von Finger 2 zu Finger 3 sind abgerundet und mit einer Körnung versehen. Die übrigen Kanten sind zwar abgerundet, besitzen aber keine Körnung. Die ungekörnte Fläche des Abstrichteils ist mit einer Stabilisierungsrippe 5 versehen, die vom Endbereich des Fingers 2 bis zum Endbereich des Fingers 3 läuft. Der längere Finger 2 weist eine gegen den anderen Finger 3 hin gerichtete polsterartige Abrundung 6 auf.

Die Grösse des Abstrichteils kann unterschiedlich sein. So kann der Arzt z.B. aus fünf Spateln wählen. Durch die gekörnte Oberfläche des Spatels ist im Sinne einer "provozierten Exfoliation" immer der Gewinn von lebenden Zellen garantiert. Ebenso ist die Zellgewinnung aus endozervikalen Bereichen von kolposkopisch stummen Prozessen möglich.

Wie Fig. 2 zeigt wird der längere Finger 2 in den Halskanal der Gebärmutter (Cervix) eingeführt. Der Spatel wird im Uhrzeigersinn gedreht, angefangen wenn der kleine Finger 3 auf drei Uhr steht, das heisst, wenn der kleine Finger 3 nach rechts zeigt. Die abgestrichenen Zellen 8 befinden sich auf der Körnung 4 und werden dann auf das Glas 7 gebracht. Beim Abstreifen der Finger 2 und 3 auf dem Glas rollen die Zellen durch die Körnung 4 hindurch und werden dabei von Schleim und anderen unerwünschten Bestandteilen gereinigt. Die Zell-Zahl im Abstrich ist um ein Vielfaches höher als bei bekannten Spateln, damit ergibt sich eine Treffsicherheit von 95 bis 97%. Die Lokalisation von Dysplasien und Karzinomen aus dem Abstrich ist möglich. Der Zellgewinn aus dem Zervikalkanal garantiert, dass auch Kulissenkarzinome immer diagnostizierbar sind.

## Ansprüche

1. Spatel für die zytologische Abstrichnahme, dadurch gekennzeichnet, dass die Oberfläche des Abstrichteils mindestens teilweise eine kantenlose Körnung (4) aufweist.

2. Spatel nach Anspruch 1, dadurch gekennzeichnet, dass die Körnung (4) halbkugelförmig bis halbovalförmig ausgebildet ist und eine Höhe von annähernd 1/10 mm aufweist.

3. Spatel nach Anspruch 1, dadurch gekennzeichnet, dass die Körnung (4) unregelmässig angeordnet ist.

4. Spatel nach Anspruch 1, dadurch gekennzeichnet, dass der bleistiftförmige Handgriffteil (1) einen kreisrunden Querschnitt und eine rauhe Oberfläche aufweist.

5. Spatel nach Anspruch 1, dadurch gekennzeichnet, dass der Abstrichteil die Form von zwei Fingern (2, 3) aufweist, die wie Zeigefinger und Daumen im Winkel voneinander gespreizt sind.

6. Spatel nach Anspruch 5, dadurch gekennzeichnet, dass der eine annähernd in Spatel-Längsrichtung zeigende Finger (2) länger und -schmäler ist als der andere Finger (3).

7. Spatel nach Anspruch 6, dadurch gekennzeichnet, dass der annähernd in Spatel-Längsrichtung zeigende Finger (2) annähernd um einen Winkel von 7° von der Spatel-Längsrichtung abweicht.

8. Spatel nach Anspruch 5, dadurch gekennzeichnet, dass der Abstrichteil mitsamt den Fingern (2, 3) flach ausgebildet ist.

9. Spatel nach Anspruch 8, dadurch gekennzeichnet, dass bei nach links gerichtetem kleineren Finger (3) die oberseitige Fläche des Abstrichteils eine Körnung (4) aufweist, die unterseitige Fläche jedoch nicht.

10. Spatel nach Anspruch 9, dadurch gekennzeichnet, dass die jeweils gegen den anderen Finger gerichteten Kanten der Finger (2, 3) sowie die Kanten des Übergangs von Finger zu Finger, abgerundet und mit einer Körnung (4) versehen sind, während die übrigen Kanten wohl abgerundet sind, aber keine Körnung aufweisen.

11. Spatel nach Anspruch 5, dadurch gekennzeichnet, dass der längere Finger (2) eine gegen den anderen Finger (3) hin gerichtete polsterartige Abrundung (6) aufweist.

12. Spatel nach Anspruch 9, dadurch gekennzeichnet, dass die ungekörnte Fläche des Abstrichteils mit einer Stabilisierungsrippe (5) versehen ist, die vom Endbereich des einen Fingers (2) bis zum Endbereich des anderen Fingers (3) läuft.

# FIG. 1

# FIG.2

# FIG.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | WO-A-8 102 974 (HASSELBRACK) * Abbildungen 5-8; Seite 10, Zeilen 18-32 * | 1,4,5 | A 61 B 10/00 |
| Y | | 6,8,11 | |
| | --- | | |
| Y | US-A-4 384 587 (MILEX PRODUCTS INC.) * Abbildungen 1,2; Zusammenfassung * | 6,8,11 | |
| | --- | | |
| A | GB-A-1 566 004 (R.N. FREDERICKS) * Abbildungen 2,4 * | 1-12 | |
| | --- | | |
| A | GB-A- 892 548 (W.B. SHUTEE) * Abbildungen 1,2 * | 1-12 | RECHERCHIERTE SACHGEBIETE (Int. Cl.4) |
| | ----- | | A 61 B |

Der vorliegende Recherchenbericht wurde für alle Patentansprüche erstellt.

| Recherchenort DEN HAAG | Abschlußdatum der Recherche 09-06-1987 | Prüfer ARGENTINI A. |
|---|---|---|

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82